Europäisches Patentamt

European Patent Office

Office européen des brevets

(19)

(11) Veröffentlichungsnummer: **0 243 606**

**A1**

(12) **EUROPÄISCHE PATENTANMELDUNG**

(21) Anmeldenummer: 87102652.2

(22) Anmeldetag: 25.02.87

(51) Int. Cl.⁴: **C12P 13/04** , C12P 13/00 , C12P 41/00

The title of the invention has been amended (Guidelines for Examination in the EPO, A-III, 7.3).

(30) Priorität: 27.02.86 DE 3606401

(43) Veröffentlichungstag der Anmeldung:
04.11.87 Patentblatt 87/45

(84) Benannte Vertragsstaaten:
BE CH DE FR GB LI NL SE

(71) Anmelder: **Gesellschaft für Biotechnologische Forschung mbH (GBF) Mascheroder Weg 1 D-3300 Braunschweig-Stöckheim(DE)**

Anmelder: **Degussa Aktiengesellschaft Degussa AG Fachbereich Patente Rodenbacher Chaussee 4 Postfach 1345 D-6450 Hanau 1 (Stadtteil Wolfgang)(DE)**

(72) Erfinder: **Sambale, Clemens, Dr. Dipl.-Chem. Mozartstrasse 22 D-6804 Ilvesheim(DE)**
Erfinder: **Kula, Maria-Regina, Dr. Dipl.-Chem. Selgenbusch 12 D-5162 Niederzier-Hambach(DE)**
Erfinder: **Hummel, Werner, Dr. Dipl.-Chem. Claudiusstrasse 11 D-5170 Titz(DE)**
Erfinder: **Drauz, Karlheinz, Dr. Rodenbacher Chaussee 4 D-6450 Hanau 1(DE)**

(74) Vertreter: **Boeters, Hans Dietrich, Dr. et al Boeters, Bauer & Partner Thomas-Wimmer-Ring 14 D-8000 München 22(DE)**

(54) Verfahren zur enzymatischen Racematspaltung.

(57) Die Erfindung betrifft ein Verfahren zur enzymatischen Spaltung von Carbamat-Racematen von Aminosäuren oder Norephedrin.

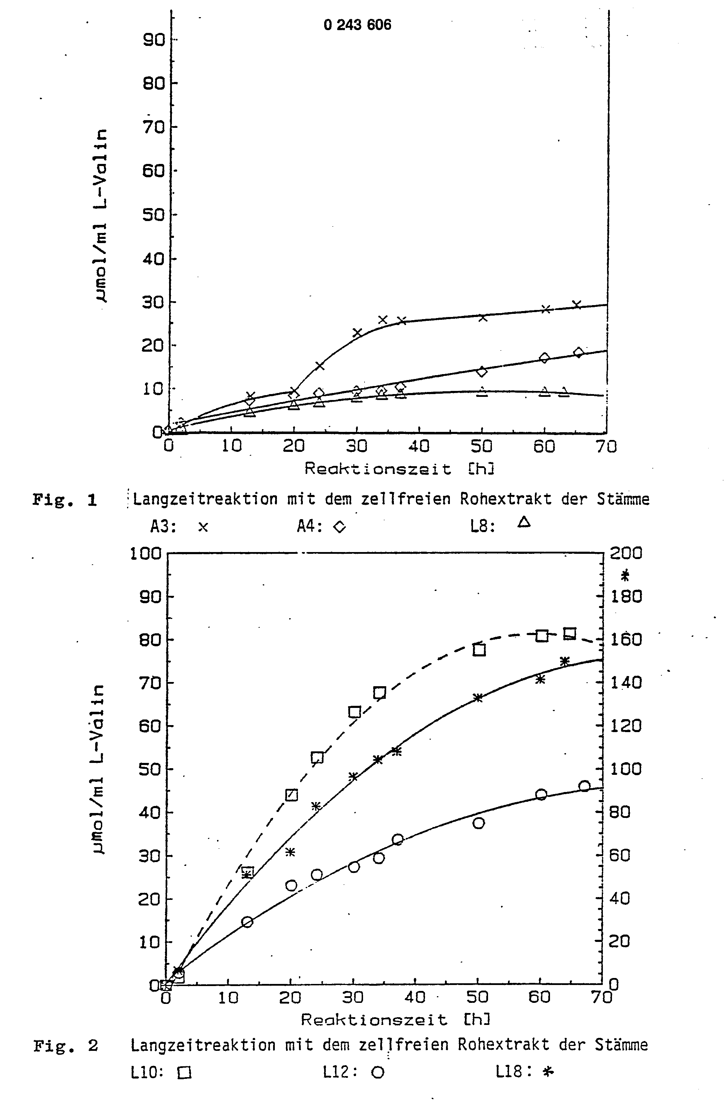

Fig. 1 Langzeitreaktion mit dem zellfreien Rohextrakt der Stämme
A3: x    A4: ◇    L8: △

## Verfahren zur Racematspaltung

Die Erfindung betrifft ein Verfahren zur Racematspaltung.

Ein ständig steigender Bedarf an Aminosäuren ist Anlaß, die vorhandenen Bezugsquellen auszubauen und zu optimieren und ferner neue Synthesewege zu erschließen. Bei den chemischen Synthesen entstehen im allgemeinen Racemate, die in weiteren Schritten in die Antipoden aufgetrennt werden müssen.

Erfindungsgemäß wird nun ein Verfahren zur Racematspaltung von Aminosäure-Derivaten vorgesehen, bei dem man von einem Aminosäurecarbamat-Racemat ausgeht und dieses Racemat enzymatisch spaltet.

Der Vorteil der Verwendung von Aminosäurecarbamaten zur Herstellung optisch aktiver Aminosäuren besteht darin, daß bei der Synthese der Ausgangsverbindungen nicht von den entsprechenden Aminosäuren ausgegangen werden muß, vielmehr Aminosäurevorstufen (beispielsweise alpha-Halogencarbonsäure) direkt zu den Carbamaten umgesetzt werden können.

Das erfindungsgemäße Verfahren zur Racematspaltung läßt sich auch auf das Norephedrincarbamat-Racemat anwenden. Norephedrin wirkt als Sympathikomimetikum.

In das erfindungsgemäße Verfahren kann man beispielsweise Methylcarbamate mit der Methoxycarbonyl-Gruppe einsetzen.

Vorteilhafterweise verwendet man ein wasserlösliches Aminosäurecarbamat-Racemat und/oder das Racemat einer in der Natur vorkommenden Aminosäure.

Man kann das erfindungsgemäße Verfahren

(a) mit Hilfe eines Mikroorganismus, der über eine Enzymaktivität verfügt, die eines der Enantiomeren eines Aminosäurecarbamat-Racemats oder Norephedrincarbamat-Racemats hydrolytisch spaltet, und der bei einem Test auf diese Enzymaktivität ermittelt worden ist, oder

(b) mit Hilfe der Enzymaktivität des Mikroorganismus gemäß (a) durchführen.

Bei der Variante (b) kann man mit Hilfe der Enzymaktivität in Form des Kulturmediums des Mikroorganismus gemäß Variante (a), des vom Mikroorganismus abgetrennten Kulturmediums, eines Extraktes aus dem Kulturmedium oder eines Extraktes aus dem Mikroorganismus spalten.

Geeignete Mikroorganismen kann man aus Bodenproben isolieren. Zur Isolierung kann man beispielsweise Bodenproben verwenden, die mit Carbamaten behandelt worden sind. Man kann sich leicht vorstellen, daß beispielsweise auf Ackerflächen, die mit Carbamatpestiziden wie Betanal oder Unden behandelt worden sind, Carbamate abbauende Mikroorganismen leben, die dort einen Selektionsvorteil genießen, so daß es zu einer Anreicherung von Stämmen mit den gesuchten Eigenschaften kommen kann.

Bei einer anderen Ausführungsform kann man das erfindungsgemäße Verfahren auch mit Hilfe eines Enzyms durchführen, das eines der Enantiomeren eines Aminosäurecarbamat-Racemates oder Norephedrincarbamat-Racemates hydrolytisch spaltet, und das bei einem Test auf diese Enzymaktivität ermittelt worden ist.

Derartige Enzyme können ein breites Gebiet neuer Anwendungen eröffnen. Dabei ist an Einsatzmöglichkeiten bei der Peptidsynthese zu denken, wo eine milde Abspaltung eingeführter Schutzgruppen, beispielsweise der t-Butyloxycarbonyl-oder Benzoxycarbonyl-Gruppe von großem Interesse ist.

Schließlich kann man gemäß einer Ausführungsform des erfindungsgemäßen Verfahrens

-die bei der Racematspaltung anfallende optisch aktive Aminosäure, insbesondere L-Aminosäure, isolieren und/oder

-das bei der Racematspaltung ferner anfallende optisch aktive Carbamat der zur anfallenden Aminosäure enantiomeren Aminosäure, insbesondere D-Aminosäure, in an sich bekannter Weise spalten und die enantiomere Aminosäure gewinnen.

In den folgenden Beispielen wird beispielsweise folgende enzymatische Racematspaltung beschrieben:

N-(Methoxycarbonyl)-DL-valin

N-(Methoxycarbonyl)-D-valin + L-Valin + $CO_2$ + Methanol

Da das $CO_2$ aus dem Gleichgewicht entfernt wird, wird dieses Gleichgewicht ständig nach rechts bzw. unten verschoben, so daß sich beide Enantiomeren in hoher Reinheit gewinnen lassen.

Nachstehend wird die Erfindung durch experimentelle Methoden und Beispiele näher erläutert.

Durchführung eines Screenings mit Bodenproben

Ein Gramm Bodenprobe wurde in 100 ml Minimalmedium (M2), welches ein Carbamatderivat als einzige C-und N-Quelle enthielt, bei 27°C und 100 Upm auf der Rundschüttelmaschine inkubiert. Nach 48h wurde mit 1% (v/v) eine weitere Kultur in dem gleichen Medium angeimpft. Zur Gewinnung von Einzelkolonien wurde mit steriler Saline* eine Verdünnungsreihe hergestellt. Aliquots wurden auf Agarplatten mit dem Minimalmedium (M2) ausgestrichen. Die Petrischalen wurden bei 27 °C 3 - 7 Tage inkubiert.

Organismen, die ein gutes Wachstum zeigten, wurden durch Verdünnungsausstrich gereinigt. Die gereinigten Stämme wurden in 100 ml Flüssigmedium (MI) in 500 ml Erlenmeyerkolben mit 2 Schikanen überimpft und bei 27 °C auf der Rundschüttelmaschine bei 100 Upm/angezogen. Nach 1-3 Tagen wurde der Kolbeninhalt abzentrifugiert. Das Sediment wurde in 0.1 M Kaliumsphophat-Puffer pH 8.0 suspendiert. Der Zellaufschluß erfolgte mit Ultraschall. Unlösliche Bestandteile wurden abzentrifugiert (30 000 g, 4 °C ). Der Überstand wurde direkt für den Enzymtest eingesetzt oder zunächst tiefgefroren (-20°C).

*Salinepuffer:

8,5 g Natriumchlorid

0,3 g $KH_2PO_4$

0,6 g $Na_2HPO_4$

10 ml 1% ige Gelatine (Merck)

990 ml $H_2O$

Medium für gescreente Stämme (MI)

Kaliumdihydrogenphosphat 0,80 g

Dikaliumhydrogenphosphat 1,76 g

Hefeextrakt 0,50 g

NaCl 0,50 g

Glucose 5,00 g

Aminosäurecarbamatderivat * 5,00 g

aufgefüllt mit dest. $H_2O$ 1,00 l pH 7.0

Medium zur Anreicherung von Mikroorganismen aus Bodenproben (M2)

Kaliumdihydrogenphosphat 0,80 g

Dikaliumhydrogenphosphat 1,76 g

Spurensalzlösung 2,00 ml

Vitaminlösung 2,00 ml

Aminosäurecarbamatderivat 10,00 g

aufgefüllt mit dest. $H_2O$ 1,00 l pH 7,0

Medium zur Fermentierung (M4)

Kaliumdihydrogenphosphat 0,80 g

Dikaliumhydrogenphosphat 1,76 g

Methanol 0,50 % (v/v)

Hefeextrakt 5,00 g

Malzextrakt 5,00 g

NaCl 3,00 g

aufgefüllt mit dest. $H_2O$ 1,00 l pH 7,00

## Kulturführung

### Schüttelkulturen

Schüttelkulturen wurden bei 30°C in 500 ml Erlenmeyerkolben (zwei Schikanen) mit 100 ml Kulturmedium auf einer Rotationsschüttelmaschine (100 Upm) angezogen. Die Animpfung erfolgte von einer Schrägagarkultur mit einer Impföse. Nach 48 h wurde von dieser ersten Vorkultur mit 1%(v/v) auf eine weitere Vorkultur überimpft. Diese diente als Vorkultur für weitere Versuchsansätze, die wiederum nach 24h mit 3 % (v/v) aus letzterer angeimpft wurden.

### Kulturführung im Fermenter (10 l Arbeitsvolumen)

Der Fermenter wurde einen Tag vor dem Animpfen sterilisiert. Bis zum Animpfen konnte somit eine Sättigung des Kulturmediums mit Sauerstoff und eine Temperaturkonstanz (30°C ±1°C) gewährleistet werden. Die Sterilisation des Fermentervolumens erfolgte in situ mit Heißdampf für die Dauer von 30 - 60 Min, 121 °C, 1,2 bar bei einer Rührgeschwindigkeit von 100 Upm. Die C-Quellen wurden ca. 1-2 h vor dem Animpfen dem Fermenteransatz aseptisch zugegeben. Zur Korrektur des pH-Wertes diente 1 M KOH bzw. 1 M $H_3PO_4$. Während des gesamten Fermentationsverlaufes wurde in der Regel gleichbleibend mit 1 vvm (Anfangswert) belüftet und mit 200 Upm gerührt.

Wenn nicht anders angegeben, wurden in den Fermentern folgende Parameter eingehalten:

Drehzahl: 200 Upm
Belüftung: 1 vvm
Temperatur: 30°C
Nährlösung: M4
pH: 7,0
Kulturdauer: 40 -80 Stunden

Während der Kulturführung wurden folgende Meßwerte permanent erfaßt:

-der Sauerstoffpartialdruck
-die Methanolkonzentration in der Abluft über einen FID
-Sauerstoff-Volumen-Konzentration der ausströmenden Reaktorluft (Vol%)
-Kohlendioxid-Volumen-Konzentration der ausströmenden Reaktorluft (Vol%)
-pH-Wert
-Kulturtemperatur
- Belüftungsrate

Zur weiteren Charakterisierung des Kulturverlaufes wurden diskontinuierlich Proben über ein sterilisierbares Ventil entnommen und für Analysen verwendet.

### Aminosäureanalysator

Die quantitative Bestimmung der Aminosäuren erfolgte auf einem Aminosäureanalysator oder im fluorimetrischen Test

Aminosäureanalysator: Biotronik LC 6000
Säule: Biotronik DC-4A (Dionex)
Vorsäule: Biotronik DC-3
Reaktionstemperatur $T_1$: 49°C, $T_2$: 63°C
Detektion: Biotronik Photometer Bt 6620 570 nm ; 440nm
Analysenzeit: 110 - 140 Min
Probenmenge: 100 µl
Eichlösung: Calbiochem - Behring Corp. Amino Acid Calibration Standard Type H

Mit dem fest eingestellten Programm wurden folgende Retentionszeiten erhalten :

Lysin 92,6 Min
α-Benzoxycarbonyl-lysin 83,2 Min
ε-Benzoxycarbonyl-lysin 82,6 Min
α-Acetyl-lysin 63,9 Min
α-Methoxycarbonyl-lysin 50,7 Min
ε-Methoxycarbonyl-lysin 48,6 Min

Methionin 48,6 Min

ε-Ethoxycarbonyl-L-lysin 48,4 Min

Valin 43,3 Min

Alanin 40,4 Min

ε-Acetyl-lysin 36,3 Min

Verdünnungspuffer/ pH 1,85: 19,60 g Na-Citrat x 2 $H_2O$

16,5 ml HCl konz.

20,0 ml Thioethanol (dest.)

0,1 ml Caprylsäure

aufgefüllt auf 5 l mit dest. $H_2O$

Trennpuffer:

Puffer A/ pH 3,20: 88,2 g Na-Citrat x 2 $H_2O$

55 - 60m HCl konz.

3,2 ml Thiodiethanol (redestilliert)

12,0 ml 25% Brij

1,0 ml Phenol

75,0 ml Ethylglycolmonomethylether

aufgefüllt auf 5 l mit dest. $H_2O$

Puffer B/pH 3,93: 88,2 g Na-Citrat x $2H_2O$

29,0 g NaCl

43,0 ml HCl konz.

12,0 ml 25% Brij

1,0 ml Phenol

aufgefüllt auf 5 l mit dest. $H_2O$

Puffer C/pH 5,00: 88,2 g Na-Citrat x $2H_2O$

29,0 g NaCl

5,0 ml HCl konz.

12,0 ml 25% Brij

1,0 ml Phenol

aufgefüllt auf 5 l mit dest. $H_2O$

Puffer D/pH 9,66: 171,5 g Na-Citrat x 2 $H_2O$

15,5 g $H_3BO_3$

1,5 g NaOH

12,0 ml 25% Brij

aufgefüllt auf 5 l mit dest. $H_2O$

Ninhydrinlösung: 3,0 l Ethylenglycolmonomethylether M 859

80,0 g Ninhydrin p. A.

1,0 l 4M Na-Acetatpuffer pH 5,51

20,0 ml Titan-III-chloridlösung 15%ig

### 5.4.3. Fluorimetrischer Test

Reagentien:

0.2 M Natriumboratpuffer pH 9,2

Fluram-Acetonlösung ( 20 mg / 100 ml )

Zu 2.25 ml Natriumboratpuffer wurden Aliquots der zu bestimmenden Aminosäurelösung (0,1 bis 100 nmol/ml) gegeben. Unter schnellem Schütteln auf einem Vortexmischer wurden 0,75 ml der Fluram-Acetonlösung zugegeben. Eine schnelle Zugabe und schnelles Mischen sind wichtig, um ein optimales Ergebnis zu erreichen. Die Fluorescenz wurde in einem Perkin-Elmer LS-5 Luminescens Gerät mit einer Anregungs - Wellenlänge von 390 nm und einer Emissions -Wellenlänge von 475 nm bis 490 nm gemessen. Von einer Eichkurve wurden die zugehörenden Werte ermittelt .

Oberprüfung der Stereospezifität

Polarimeter

Die Messungen wurden am Perkin-Elmer-Polarimeter Typ 241 bei 436 nm, 25°C durchgeführt. Es wurden Eichreihen im Bereich von 0-300 mM aufgenommen, mit der quantitativ die Aminosäurekonzentration bestimmt werden konnte.

## Definitionen :

$$\text{spezifische Drehung } (\alpha) = \frac{\alpha}{c \times l}$$

$$\text{optische Reinheit } \quad p = \frac{\alpha}{\alpha_{max}}$$

$( \alpha )$ : spezifische Drehung $\qquad \alpha$ : Drehung

c: Konzentration g/l $\qquad l$ : Länge (dm)

L-Aminosäureoxideasetest L-Aminosäureoxidase aus Crotalus durissus wurde zum Test auf L-Aminosäuren eingesetzt. Der Test wurde nach der Vorschrift von Boehringer Mannheim durchgeführt.

Lösungen:

a) Triethanolaminpuffer 0,2 M , pH 7,6
b) o-Dianisidin Lösung 23,2 mM
c) Puffer - Aminosäurelösung + 0,5 ml Lösung (b).
d) Peroxidase (POD) 250 U/mg
e) Enzymlösungen U/mg 1/100 oder 1/50 oder 1/20 mit destilliertem, eisgekühltem Wasser verdünnt.

Reaktionsansatz:

Pufferlösung (c) 3,00 ml
POD Suspension (d) 0,01 ml
Enzymlösung (e) 0,02 ml
Die Reaktion wurde bei 436 nm und 25 °C an einem DU-Beckmann-Spektralphotometer durchgeführt. Eine Eichreihe mit L-Alanin und L-Valin zeigte einen linearen Zusammenhang im Bereich von 40 bis 400 µM.

## D-Aminosäureoxidasetest

D-Aminosäureoxidase aus Schweineniere wurde zum Test auf D-Aminosäuren eingesetzt. Der Test wurde nach der Vorschrift der Firma Boehringer Mannheim durchgeführt.

Lösungen:

a) Tris-Puffer, 200 mM pH 8,3 10 min mit $O_2$ begast
b) NADH Lösung, 12 mM ( 10 mg NADH , Na-Salz / ml $H_2O$)
c) Katalase - Lösung: ca. 260 000 U/ml in destilliertem Wasser 1:100 verdünnt.
d) Lactatdehydrogenase (LDH) ungefähr 450 U / mg
e) Enzymlösungen, ungefähr 15 U/mg 1/50 , 1/20 , 1/10 verdünnt mit eiskaltem Wasser.

Eine Eichreihe mit D-Alanin und D-Valin zeigte einen linearen Zusammenhang von 10 bis 350 $\mu$M. Die Nachweisgrenze lag bei 5 $\mu$M D-Aminosäure.

Reaktionsansatz:

Trispuffer (a) 2,50 ml
Aminosäuretestlösung 0,50 ml
NADH Lösung (b) 0,05 ml
Catalase Lösung(c) 0,01 ml
LDH Suspension(d) 0,01 ml
Enzymlösung(e) 0,01 ml

Die Änderung der Lichtabsorption bei 340 nm wurde bei 25 °C spektralphotometrisch gemessen.

## Beispiele 1 bis 42

Bei der Suche nach Mikroorganismen mit stereoselektiv Aminosäurecarbamate hydrolysierenden Enzymen wurden Bodenproben von unterschiedlichen Standorten eingesetzt, um ein möglichst breites Spektrum an Mikroorganismen isolieren zu können.

Die Proben wurden auf einem Bauernhof und auf mit den Carbamatpestiziden Betanal 32 und Unden 33 behandelten Ackerflächen entnommen.

Das Screeningergebnis zeigte keinen besonders zu bevorzugenden Fundort. Die aus dem Boden ausgeschwemmten Mikroorganismen wurden auf N-(Methoxycarbonyl)-DL-alanin, N-(Methoxycarbonyl)-DL-valin oder N-alpha-epsilon-(Dimethoxycarbonyl)-DL-lysin als jeweils einziger C-und N-Quelle angezogen. Auf N-(Methoxycarbonyl)-DL-valin wurden deutlich weniger Stämme gefunden, als auf den beiden anderen Aminosäurecarbamaten.

Von 42 gefundenen Stämmen zeigten 35 Stämme die höchste Enzymaktivität bei der Umsetzung des Substrates N-(Methoxycarbonyl)-DL-alanin. Die enzymatische Hydrolyse von N-alpha-epsilon-)Dimethoxycarbonyl)-DL-lysin kann theoretisch zu vier Verbindungen führen, dem D-oder dem L-Enantiomer des N-epsilon-(Methoxycarbonyl)-lysins, dem N-alpha-(Methoxycarbonyl)-DL-lysin und dem Lysin.

Alle Stämme, die Aktivität bei der Umsetzung von N-alpha-epsilon-(Dimethoxycarbonyl)-DL-lysin zeigten, bildeten das Produkt N-epsilon-(Methoxycarbonyl)-lysin, von dem in einigen Fällen das L-Enantiomer nachgewiesen werden konnte. Bei 5 von 42 Stämmen konnte auch die freie Aminosäure Lysin nachgewiesen werden. Die Verbindung N-alpha-(Methoxycarbonyl)-DL-lysin konnte in keinem Fall nachgewiesen werden. Die Enzyme greifen somit bevorzugt die alpha-Stellung im Lysindicarbamat an.

Es fällt auf, daß alle näher untersuchten Wildtypisolate die erwartete Stereoselektivität in der Hydrolyse der racemischen Aminosäurecarbamate zeigen.

Die Ergebnisse von Langzeitreaktionen mit 6 der 42 gefundenen Stämme sind Tabelle 1 und den Figuren 1 und2 zu entnehmen.

Tabelle 1

Langzeitreaktion von zellfreiem Rohextrakt mit dem Substrat N-(Methoxycarbonyl)-DL-valin

| Stamm | Protein mg/ml | Substrat mmol/40ml | Ink.- zeit/h | Produkt mmol/40ml | Produkt mg/40ml | Ausbeute % |
|-------|------|------|------|------|------|------|
| A4  | 1,2 | 7,0  | 65,0 | 1,18 | 138,2 | 33,71 |
| A3  | 0,2 | 7,0  | 65,5 | 0,74 | 86,6  | 21,14 |
| L8  | 1,5 | 7,0  | 63,5 | 0,37 | 44,7  | 10,74 |
| L10 | 0,5 | 7,0  | 67,0 | 1,84 | 215,8 | 52,65 |
| L12 | 4,0 | 7,0  | 64,0 | 2,99 | 350,2 | 85,43 |
| L18 | 1,4 | 14,0 | 64,5 | 6,50 | 761,4 | 92,86 |

Die Stereospezifität der Biotransformation mit den sechs Mikroorganismenstämmen wurde auch dadurch bewiesen, daß die Verbindung N-(Methoxycarbonyl)-D-valin nicht umgesetzt wurde. Auffallend ist der Aktivitätsverlauf von Stamm A3. Da es sich um Rohextrakte handelte, können eine Vielzahl von Faktoren, z.B. Inhibitoren, Einfluß auf den Reaktionsverlauf haben.

### Beispiele 43 bis 50

Die hydrolytische Umsetzung der Aminosäurecarbamate zur Aminosäure, Kohlendioxid und Alkohol kann durch einen enzymatischen Angriff auf die Amidbindung oder Esterbindung bewirkt werden. Diese Überlegungen führten zu der Frage, inwieweit käufliche Enzyme, die Ester-und Amidbindungen spalten, Aminosäurecarbamate als Substrate akzeptieren. Es wurden eine Esterase, drei Acetylcholinesterasen, eine Hydantoinase, eine Urease und zwei Acylasen untersucht.

Carboxylesterase (Beispiel 43) aus Schweineleber zeigte keine Reaktion. Während die Acetylcholinesterase vom Aal (Beispiel 44) die Aminosäurecarbamate nicht umsetzt, konnte Enzymaktivität bei der Acetylcholinesterase aus Erythrozyten des Rindes (Beispiel 45) und des Menschen (Beispiel 46) gefunden werden. Es wurden nur die L-Aminosäurecarbamate umgesetzt. Die D-Antipode als Substrat eingesetzt, zeigte keine Reaktion.

Die Enzyme zeigten eine gute Stabilität. In Inkubationsansätzen über einen Zeitraum von 150 Stunden wurde ein linearer Substratumsatz erreicht. Die Hydantoinase aus Pseudomonas fluorescens (Beispiel 47) zeigte keine Aktivität. Keine Aktivität wurde ebenfalls bei der Urease (Beispiel 48) gefunden. Die gesuchte Aktivität konnte auch bei der Pilzacylase (Beispiel 49) und der Acylase aus Schweinenieren (Beispiel 50) nachgewiesen werden.

Tabelle 2 zeigt einen Vergleich der prozentualen Umsatzrate von Acetyl-und Carbamataminosäuren mit der Nierenacylase. Die Reaktionen waren L-spezifisch.

Tabelle 2

Substratspezifität von Aminoacylase aus Schweineniere (auf Alanin (100 %) bezogen).

| Acylaminosäuren | | | Aminosäurecarbamate | |
| --- | --- | --- | --- | --- |
| Aminosäure | $-\underset{O}{\overset{\parallel}{C}}-R$ | | $-\underset{O}{\overset{\parallel}{C}}-OR$ | relative Umsatzrate |
| Alanin | Chloracetyl | 100 | Methoxycarbonyl | 100 |
| | Acetyl | 27 | Ethoxycarbonyl | 89 |
| Valin | Acetyl | 14,5 | Methoxycarbonyl | 71 |
| | | | Ethoxycarbonyl | 59 |

**Ansprüche**

1. Verfahren zur Racematspaltung, dadurch **gekennzeichnet,** daß man von einem Carbamat-Racemat einer Aminosäure oder des Norephedrins ausgeht und dieses Racemat enzymatisch spaltet.

2. Verfahren nach Anspruch 1, dadurch **gekennzeichnet,** daß man ein Methylcarbamat verwendet.

3. Verfahren nach Anspruch 1 oder 2, dadurch **gekennzeichnet,** daß man ein wasserlösliches Aminosäurecarbamat-Racemat verwendet.

4. Verfahren nach einem der vorhergehenden Ansprüche, dadurch **gekennzeichnet,** daß man das Aminosäurecarbamat-Racemat einer in der Natur vorkommenden Aminosäure verwendet.

5. Verfahren nach einem der vorhergehenden Ansprüche, dadurch **gekennzeichnet,** daß man das Verfahren

(a) mit Hilfe eines Mikroorganismus, der über eine Enzymaktivität verfügt, die eines der Enantiomeren eines Aminosäurecarbamat-Racemats oder Norephedrincarbamat-Racemats hydrolytisch spaltet, und der bei einem Test auf diese Enzymaktivität ermittelt worden ist, oder

(b) mit Hilfe der Enzymaktivität des Mikroorganismus gemäß (a)

durchführt.

6. Verfahren nach Anspruch 5 (b), dadurch **gekennzeichnet,** daß man mit Hilfe der Enzymaktivität in Form des Kulturmediums des Mikroorganismus gemäß Anspruch 5 (a), des vom Mikroorganismus abgetrennten Kulturmediums, eines Extraktes aus dem Kulturmedium oder eines Extraktes aus dem Mikroorganismus spaltet.

7. Verfahren nach einem der vorhergehenden Ansprüche, dadurch **gekennzeichnet,** daß man enzymatisch mit Hilfe eines Mikroorganismus oder der Enzymaktivität eines Mikroorganismus spaltet, den man aus einer Bodenprobe isoliert hat.

8. Verfahren nach Anspruch 7, dadurch **gekennzeichnet,** daß man enzymatisch mit Hilfe eines Mikroorganismus oder der Enzymaktivität eines Mikroorganismus spaltet, den man aus einer Bodenprobe isoliert hat, die mit Carbamaten behandelt worden ist.

9. Verfahren nach einem der Ansprüche 1 bis 4, dadurch **gekennzeichnet,** daß man das Verfahren mit Hilfe eines Enzyms durchführt, das eines der Enantiomeren eines Aminosäurecarbamat-Racemates oder Norephedrincarbamat-Racemats hydrolytisch spaltet und das bei einem Test auf diese Enzymaktivität ermittelt worden ist.

10. Verfahren nach einem der vorhergehenden Ansprüche, dadurch **gekennzeichnet,** daß man
-die bei der Racematspaltung anfallende optisch aktive Aminosäure, insbesondere L-Aminosäure, isoliert und/oder
-das bei der Racematspaltung ferner anfallende optisch aktive Carbamat der dazu enantiomeren Aminosäure, insbesondere D-Aminosäure, in an sich bekannter Weise spaltet und die enantiomere Aminosäure gewinnt.

0 243 606

Fig. 1   Langzeitreaktion mit dem zellfreien Rohextrakt der Stämme

A3: ×          A4: ◇          L8: △

Fig. 2   Langzeitreaktion mit dem zellfreien Rohextrakt der Stämme

L10: □          L12: ○          L18: *

| | Europäisches Patentamt | **EUROPÄISCHER RECHERCHENBERICHT** | Nummer der Anmeldung |
| | | | EP 87 10 2652 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | FR-A-2 367 717 (HOFFMANN-LA ROCHE) | 1 | C 12 P 13/04<br>C 12 P 13/00<br>C 12 P 41/00 |
| Y | CHEMICAL ABSTRACTS, Band 85, Nr. 5, 2. August 1976, Seite 144, Zusammenfassung Nr. 29884g, Columbus, Ohio, US; J.C. BAKER et al.: "The enzymic hydrolysis of N-carbethoxyproline and N-carbethoxyglycine", & AM. J. ENOL. VITIC. 1976, 27(1), 12-14 | 1 | |
| A | CHEMICAL ABSTRACTS, Band 97, Nr. 19, 8. November 1982, Seite 349, Zusammenfassung Nr. 158940u, Columbus, Ohio, US; L. PINHEIRO-DA-SILVA FILHO et al.: "Papain-catalyzed esterification of N-tert-butyloxycarbonyl and N-benzyloxycarbonyl amino acids", & BRAZ. J. MED. BIOL. RES. 1981, 14(6), 373-7 | 1 | |

RECHERCHIERTE SACHGEBIETE (Int. Cl.4)

C 12 P

Der vorliegende Recherchenbericht wurde für alle Patentansprüche erstellt.

| Recherchenort | Abschlußdatum der Recherche | Prüfer |
|---|---|---|
| DEN HAAG | 20-05-1987 | DELANGHE L.L.M. |